**Europäisches Patentamt**

**European Patent Office**

**Office européen des brevets**

(19)

(11) Veröffentlichungsnummer : **0 401 864 B1**

(12)

# EUROPÄISCHE PATENTSCHRIFT

(45) Veröffentlichungstag der Patentschrift :
**04.11.92 Patentblatt 92/45**

(51) Int. Cl.$^5$ : **A61F 2/66**

(21) Anmeldenummer : **90110970.2**

(22) Anmeldetag : **09.06.90**

(54) **Gelenkloser Prothesenfuss.**

(30) Priorität : **09.06.89 DE 3918810**

(43) Veröffentlichungstag der Anmeldung :
**12.12.90 Patentblatt 90/50**

(45) Bekanntmachung des Hinweises auf die
Patenterteilung :
**04.11.92 Patentblatt 92/45**

(84) Benannte Vertragsstaaten :
**AT BE CH DE DK ES FR GB IT LI NL SE**

(56) Entgegenhaltungen :
**EP-A- 0 331 468**
**WO-A-88/00815**
**DE-C- 311 463**
**DE-C- 325 171**

(56) Entgegenhaltungen :
**FR-A- 2 293 186**
**US-A- 2 440 075**
**US-A- 4 547 913**
**US-A- 4 645 509**

(73) Patentinhaber : **Otto Bock Orthopädische**
**Industrie Besitz- und**
**Verwaltungs-Kommanditgesellschaft**
**Industriestrasse**
**W-3408 Duderstadt 1 (DE)**

(72) Erfinder : **Näder, Max, Dr.-Ing.**
**Hindenburgring 39**
**W-3408 Duderstadt (DE)**

(74) Vertreter : **Gramm, Werner, Prof. Dipl.-Ing. et al**
**Patentanwälte Gramm + Lins**
**Theodor-Heuss-Strasse 2**
**W-3300 Braunschweig (DE)**

**Beschreibung**

Die Erfindung betrifft einen gelenklosen Prothesenfuß mit einem innerhalb des Prothesenkörpers vorgesehenen, die Prothesenbelastungen aufnehmenden und übertragenden federelastischen Fußeinsatz, der einen oberen horizontalen, den oberen Abschluß des Prothesenfußes bildenden und eine Verbindungsmöglichkeit mit der Prothese bietenden Schenkel sowie einen unteren Schenkel aufweist, der gegenüber dem oberen Schenkel verlängert ausgebildet ist und sich mit seinem freien Ende bis in den Fußspitzenbereich hinein erstreckt zur Erzielung einer hohen Vorfußelastizität in Verbindung mit einem hohen Energiespeichervermögen bei Vorfußbelastung.

Eine entsprechende Ausführungsform ist durch den sogenannten Seattle-Fuß bekanntgeworden (siehe VETERANS ADMINISTRATION - JOURNAL OF REHABILITATION RESEARCH AND DEVELOPMENT, Bd. 22, Nr.3 BPR 10-42, Seiten 75-84, insbesondere Abbildung 6 und US-A-4 645 509).

Die Funktion eines Prothesenfußes hängt im wesentlichen von seinen elastischen Eigenschaften ab. Diese werden bestimmt durch Material, Gestalt und Anordnung der verwendeten elastischen Komponenten.

Je nach vorgesehenem Einsatzbereich, der sich je nach Neigungen des Amputierten vom normalen Gehen in der Ebene bis zum sportlichen Einsatz wie Joggen, Laufen, Springen erstrecken kann, sind die Anforderungen an solche Komponenten sehr unterschiedlich, insbesondere was ihr Arbeitsvermögen und der eng damit im Zusammenhang stehende Verlauf ihrer Federkennlinien bei Be- und Entlastung betrifft.

Die jüngeren Entwicklungen gelenkloser Prothesenfüße für einen möglichst weiten Einsatzbereich weichen vom klassischen Konzept des SACH-Fußes dadurch ab, daß sie den starren Fußkern im Vorfußbereich mit Federelementen ergänzen oder ihn ganz durch solche ersetzen (z.B. SEATTLE-Fuß), um die Vorfußelastizität und damit auch das Energiespeichervermögen bei Vorfußbelastung zu verbessern. Die Fersenelastizität wird dabei konzeptionell weitgehend unverändert durch einen Schaumstoffkeil im Fersenbereich erreicht.

Der Erfindung liegt die Aufgabe zugrunde, einen Prothesenfuß der eingangs erläuterten Ausführungsart mit verbesserten Eigenschaften zu entwickeln.

Diese Aufgabe wird gemäß der Erfindung dadurch gelöst, daß der zumindest eine Plantar- und Dorsalflexion sowie eine axiale Kompression ermöglichende Fußeinsatz im Fußlängsschnitt eine angenähert Z-förmige Ausbildung aufweist, wobei der genannte obere Schenkel sowie der genannte untere Schenkel mit dem schrägen Z-Balken einen oberen bzw. unteren Winkel ($\alpha$ , $\beta$) einschließen und einen hinteren bzw. vorderen keilförmigen Freiraum begrenzen.

Dabei ist es vorteilhaft, wenn der obere Schenkel des Fußeinsatzes in seiner Horizontalebene gegenüber dem unteren Schenkel verwindbar ist, also eine Torsion um eine vertikale Achse zuläßt, und wenn darüber hinaus der obere Schenkel des Fußeinsatzes in der lotrecht und quer zur Gehrichtung liegenden Frontalebene gegenüber dem unteren Schenkel verwindbar ist zur Anpassung an Neigungen quer zur Gehrichtung.

Erfindungsgemäß ist es möglich, wenn die die Kraft-Weg- bzw. Winkel-Moment-Charakteristik der genannten Verschwenkungen bzw. Verformungen bestimmenden federelastischen Eigenschaften (Federkennlinien) durch den Z-förmigen Fußeinsatz allein bestimmt sind. Es ist aber auch möglich, daß die genannten Federkennlinien im Zusammenwirken mit dem Z-förmigen Fußeinsatz mit zusätzlichen federelastischen Komponenten erzielt werden, die in zumindest einem der beiden genannten keilförmigen Freiräume angeordnet sind.

Erfindungsgemäß ist es grundsätzlich möglich, den Fußeinsatz ungegliedert einstückig auszubilden. Er kann erfindungsgemäß aber auch zweigliedrig oder dreigliedrig ausgeführt werden. Der Fußeinsatz bzw. seine einzelnen Glieder können eine Monostruktur oder eine geschichtete Struktur, z.B. als Blattfederstruktur aufweisen bzw. aus einem einheitlichen Werkstoff oder aber mehreren verschiedenen Materialien bestehen. Auch bei einer gegliederten Ausführung des Z-förmigen Fußeinsatzes ist diese Gliederung von außen nicht erkennbar, so daß der erfindungsgemäße Prothesenfuß auch weiterhin die kosmetischen Vorteile eines gelenklosen Fußes bietet.

Die Plantarflexion wird durch Verkleinerung des oberen Winkels $\alpha$ in einer Ebene senkrecht zum Becken und parallel zur Laufrichtung erreicht. Die genannte Dorsalflexion, ergibt sich durch Verkleinerung des entsprechenden unteren Winkels $\beta$. Eine Verkleinerung beider Winkel $\alpha$ und $\beta$ führt zur axialen Kompression, also zu einer Verformung/Bewegung senkrecht zur Bodenebene.

Bewegungen im Sinne einer Vergrößerung der Winkel $\alpha$ und/oder $\beta$ werden entweder durch die Struktursteifigkeit des Z-förmigen Fußeinsatzes allein und/oder durch eine zusätzliche Einrichtung begrenzt. Diese zusätzliche Einrichtung kann z.B. ein auf den Z-förmigen Fußeinsatz aufgezogener, sockenähnlich ausgebildeter Überzug sein. Es könnten aber auch mechanische Anschläge und/oder flexible Gurtungen o.dergl. vorgesehen werden.

Erfindungsgemäß können alle Funktionselemente in Fußform umschäumt und/oder von einer kosmetischen Hülle umschlossen sein.

Der obere horizontale Schenkel des Fußeinsatzes ist so gestaltet, daß er eine separate oder integrierte Verbindungsmöglichkeit mit der Prothese bietet.

Eine besondere Beeinflussung der Federkennlinien des Prothesenfußes kann dadurch erreicht werden, daß separate oder integrierte Stützkonturen an dem Z-förmigen Fußeinsatz vorgesehen sind. Diese Stützkonturen können hart oder hartelastisch ausgebildet sein und sind vorzugsweise so geformt, daß sich durch sie ein Anlagepunkt zwischen zwei Schenkeln des Z-förmigen Fußeinsatzes bei zunehmender Belastung verlagert. Insbesondere können die Stützkonturen zwischen dem unteren Schenkel und dem schrägen Z-Balken vorgesehen und so ausgebildet sein, daß sie sich bei Belastung berühren und der Anlagepunkt bei zunehmender Belastung nach vorne wandert, so daß sich die für den unteren Schenkel zur Verfügung stehende freie Federlänge verkürzt. Dadurch entsteht bei zunehmender Belastung eine progressiv härtere Federkennlinie für den unteren horizontalen Schenkel.

Weitere Merkmale der Erfindung sind. Gegenstand der Unteransprüche und werden in Verbindung mit weiteren Vorteilen der Erfindung anhand von Ausführungsbeispielen näher erläutert.

In der Zeichnung sind einige als Beispiele dienende Ausführungsformen der Erfindung schematisch jeweils im Längsschnitt dargestellt. Es zeigen:

Figur 1 einen Prothesenfuß mit einem ungegliederten einstückigen Fußeinsatz;

Figur 2 einen Prothesenfuß mit einem zweigliedrig ausgebildeten Fußeinsatz;

Figur 3 einen Prothesenfuß mit einem dreigliedrig ausgebildeten Fußeinsatz;

Figur 4 einen Prothesenfuß mit einem Fußeinsatz gemäß Figur 1, dessen hinterer und vorderer keilförmige Freiraum mit einem federelastischen Stoff ausgefüllt ist;

Figur 5 einen Prothesenfuß mit einem Fußeinsatz gemäß Figur 3, in dessen keilförmigen Freiräumen Druckfedern angeordnet sind;

Figur 6 einen Prothesenfuß mit einem Fußeinsatz gemäß Figur 1, der mit zusätzlichen Stützkonturen versehen ist und

Figur 7 eine Fußprothese mit einem Fußeinsatz gemäß Figur 1 mit einem sockenartigen Überzug.

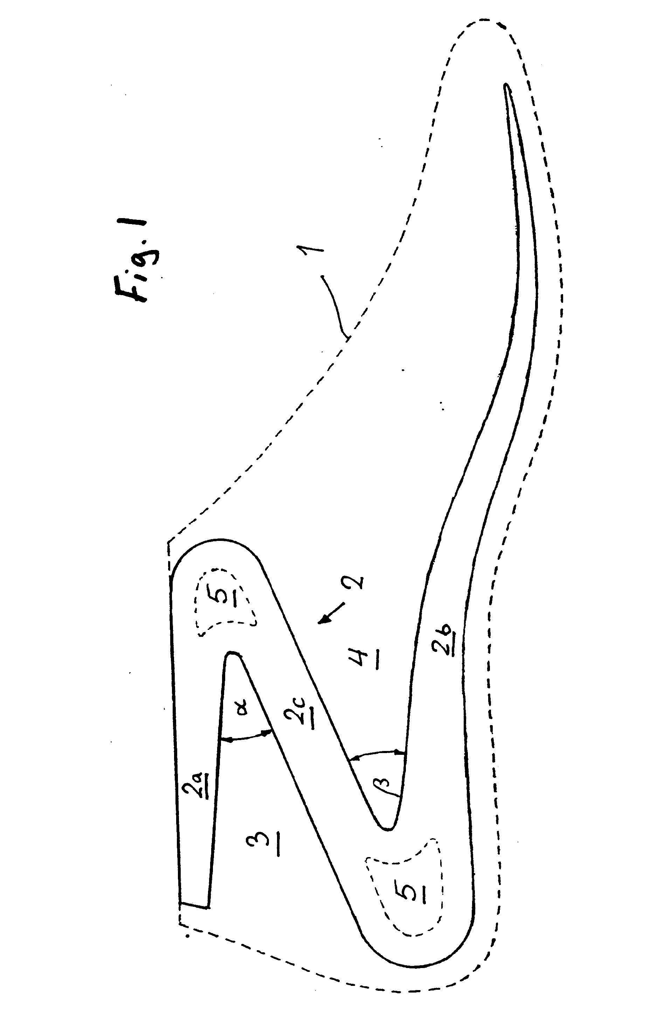

Figur 1 zeigt einen gelenklosen Prothesenfuß mit einem innerhalb eines gestrichelt dargestellten Prothesenkörpers 1 vorgesehenen, die Prothesenbelastungen aufnehmenden und übergebenden federelastischen Fußeinsatz 2, der im Fußlängsschnitt eine angenähert Z-förmige Ausbildung aufweist. Der obere Schenkel 2a sowie der untere Schenkel 2b schließen mit dem schrägen Z-Balken 2c einen oberen Winkel $\alpha$ bzw. einen unteren Winkel $\beta$ ein und begrenzen einen hinteren keilförmigen Freiraum 3 sowie einen vorderen keilförmigen Freiraum 4. Der obere horizontale Schenkel 2a bildet den oberen Abschluß des Prothesenfußes und bietet eine Verbindungsmöglichkeit mit der nicht näher dargestellten Prothese. Der untere Schenkel 2b ist ebenfalls angenähert horizontal ausgerichtet, ist gegenüber dem oberen Schenkel 2a verlängert ausgebildet und erstreckt sich mit seinem freien Ende bis in den Fußspitzenbereich hinein. Eine etwaige Neigung des unteren Schenkels 2b ergibt sich aus der vorgesehenen Absatzhöhe.

Der Z-förmige Fußeinsatz 2 ergibt eine hohe Vorfußelastizität in Verbindung mit einem hohen Energiespeichervermögen bei Vorfußbelastung. Ferner läßt der Fußeinsatz 2 eine Plantar- und Dorsalflexion sowie eine axiale Kompression zu. Darüber hinaus ist der obere Schenkel 2a des Fußeinsatzes 2 in seiner Horizontalebene gegenüber dem unteren Schenkel 2b verwindbar, läßt also eine Torsion um eine vertikale Achse zu. Darüber hinaus ist der obere Schenkel 2a in der lotrecht und quer zur Gehrichtung liegenden Frontalebene gegenüber dem unteren Schenkel 2b verwindbar, so daß sich eine Anpassung an Neigungen quer zur Gehrichtung ergibt. Die untere Kontur des unteren horizontalen Schenkels 2b ist an die Kontur der Fußunterseite angepaßt.

Die gestrichelt eingezeichneten Bereiche 5 deuten Hohlräume an, die zur Vermeidung von Materialansammlungen vorgesehen werden können.

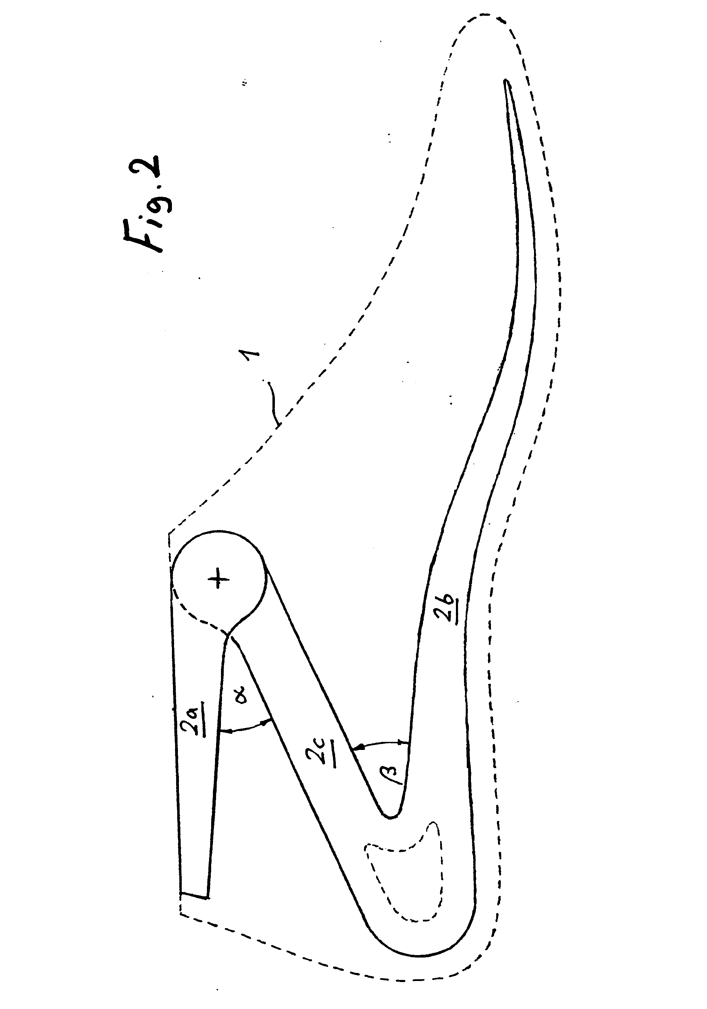

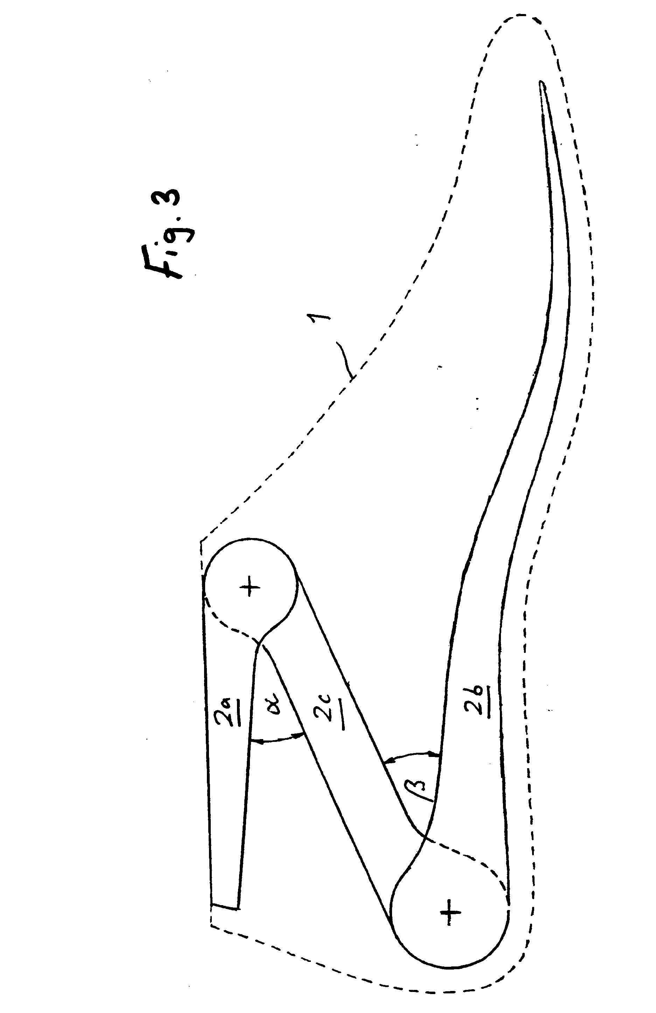

Während Figur 1 einen ungegliederten einstückigen Fußeinsatz 2 zeigt, ist in Figur 2 ein zweigliedrig ausgebildeter Fußeinsatz 2 dargestellt, dessen oberer Schenkel 2a an dem oberen Ende des schrägen Z-Balkens 2c ggf. gelenkig verbunden ist. Figur 3 zeigt einen dreigliedrig ausgebildeten Fußeinsatz 2, dessen beiden Schenkel 2a, 2b an dem schrägen Z-Balken 2c ggf gelenkig verbunden sind.

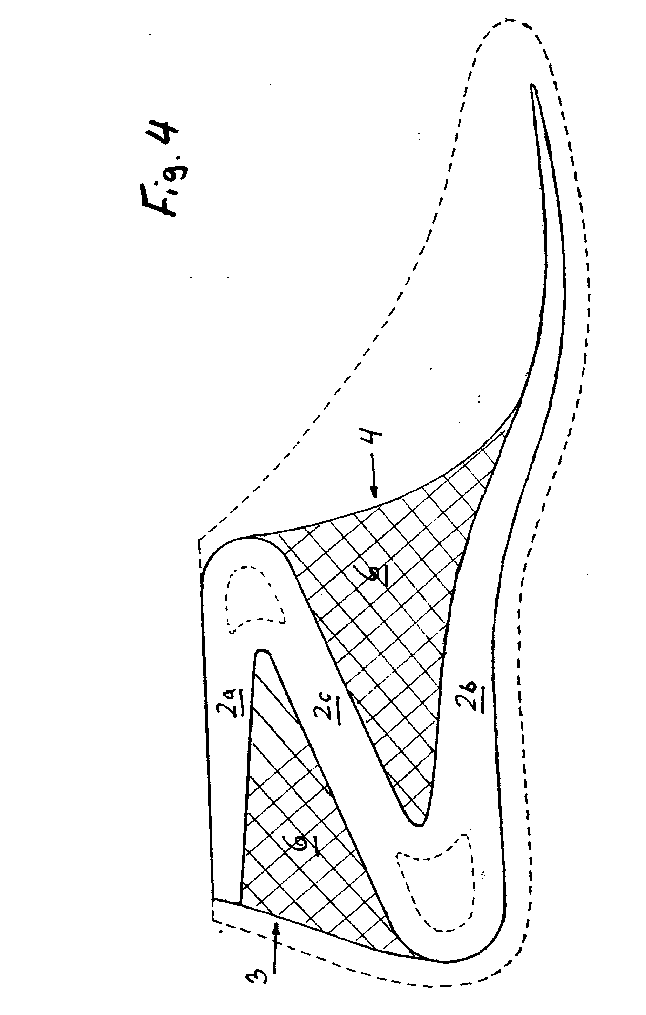

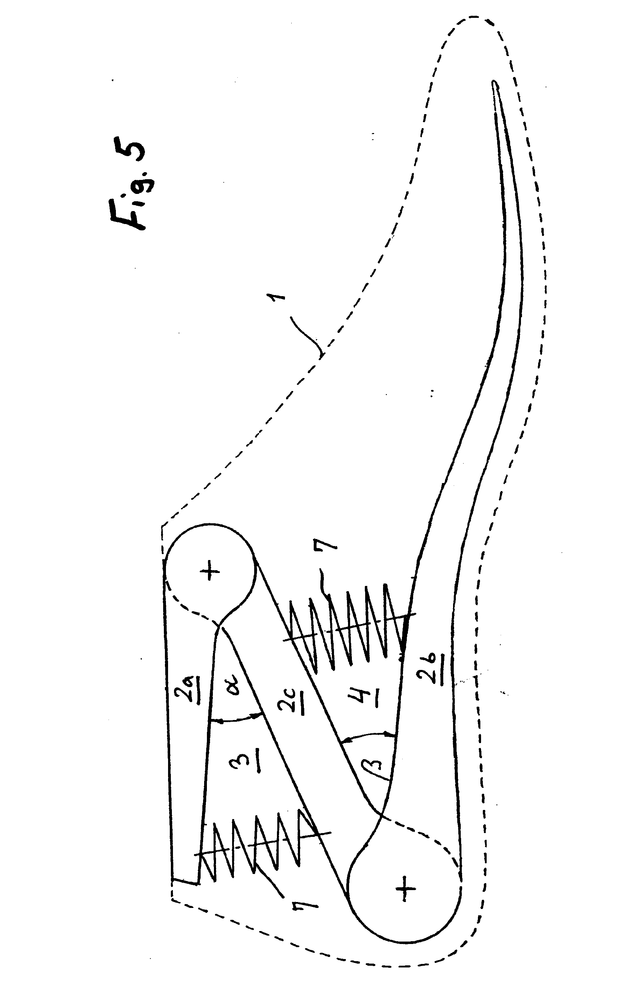

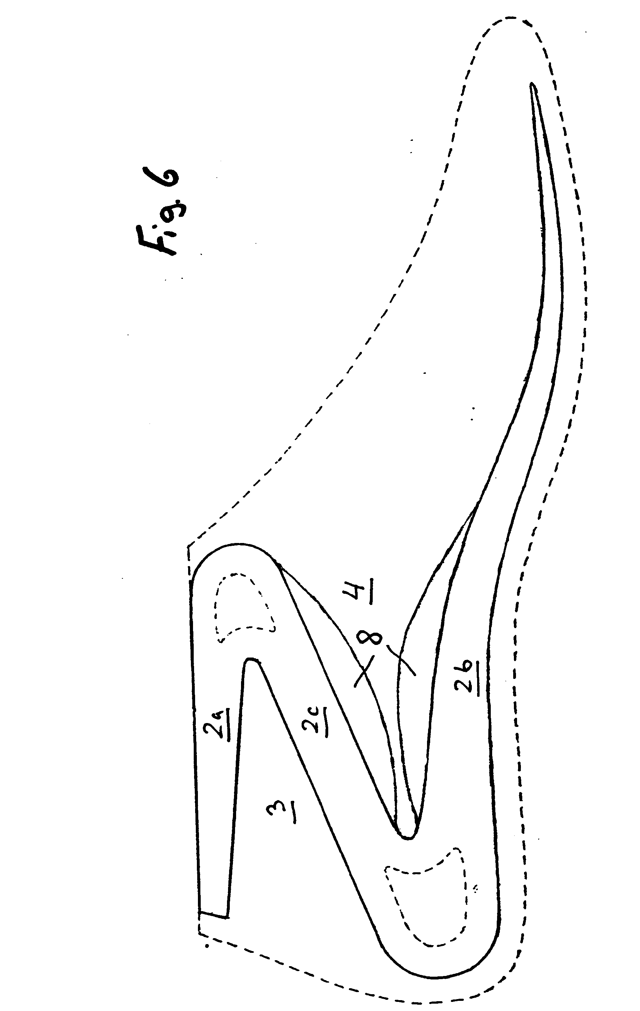

Während bei den in den Figuren 1 bis 3 dargestellten Ausführungsformen die Federkennlinien durch den Z-förmigen Fußeinsatz 2 allein bestimmt sind, zeigen die Figuren 4, 5 und 6 verschiedene Ausführungsbeispiele dafür, daß die genannten Federkennlinien im Zusammenwirken mit dem Z-förmigen Fußeinsatz 2 durch zusätzliche federelastische Komponenten erzielt werden, die in zumindest einem der beiden keilförmigen Freiräume 3,4 angeordnet sind. Bei dem Beispiel gemäß Figur 4 sind die zusätzlichen federelastischen Komponenten durch einen federelastischen Stoff 6 gebildet, der die beiden Freiräume 3,4 ausfüllt. Bei dem Ausführungsbeispiel gemäß Figur 5 sind die zusätzlichen federelastischen Komponenten Druckfedern 7, während bei dem Lösungsvorschlag gemäß Figur 6 die zusätzlichen federelastischen Komponenten durch separate oder integrierte Stützkonturen 8 an dem schrägen Z-Balken 2c sowie an dem unteren Schenkel 2b des Fußeinsatzes 2 sind. Diese Stützkonturen 8 sind im vorderen keilförmigen Freiraum 4 angeordnet.

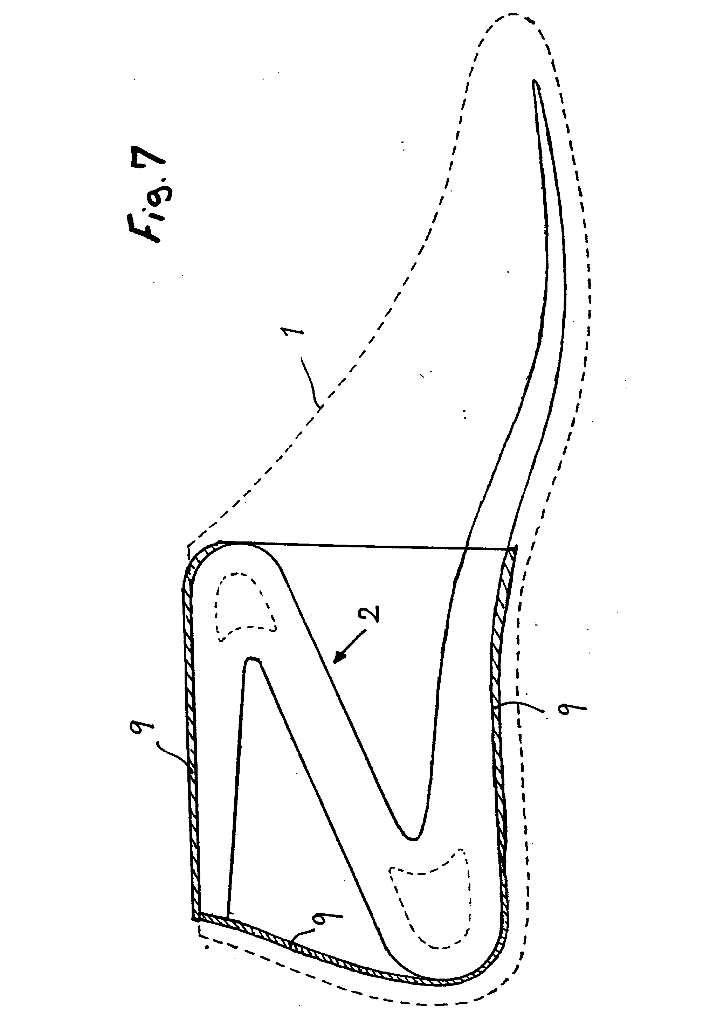

Figur 7 zeigt eine Ausführungsform gemäß Figur 1, wobei jedoch der Fußeinsatz 2 mit einem sockenähnlichen Überzug 9 versehen ist, durch den der Verschwenkbereich der beiden Schenkel 2a,2b des Z-förmigen Fußeinsatzes 2 im Sinne einer Vergrößerung des oberen Winkels α bzw. des unteren Winkels β begrenzt wird.

## Patentansprüche

1. Gelenkloser Prothesenfuß mit einem innerhalb des Prothesenkörpers (1) vorgesehenen, die Prothesenbelastungen aufnehmenden und übertragenden federelastischen Fußeinsatz (2), der einen oberen horizontalen, den. oberen Abschluß des Prothesenfußes bildenden und eine Verbindungsmöglichkeit mit der Prothese bietenden Schenkel (2a) sowie einen unteren Schenkel (2b) aufweist, der gegenüber dem oberen Schenkel (2a) verlängert ausgebildet ist und sich mit seinem freien Ende bis in den Fußspitzenbereich hinein erstreckt zur Erzielung einer hohen Vorfußelastizität in Verbindung mit einem hohen Energiespeichervermögen bei Vorfußbelastung, **dadurch gekennzeichnet,** daß der zumindest eine Plantar- und Dorsalflexion sowie eine axiale Kompression ermöglichende Fußeinsatz (2) im Fußlängsschnitt eine angenähert Z-förmige Ausbildung aufweist, wobei der genannte obere Schenkel (2a) sowie der genannte untere Schenkel (2b) mit dem schrägen Z-Balken (2c) einen oberen bzw. unteren Winkel (α , β) einschließen und einen hinteren bzw. vorderen keilförmigen Freiraum (3,4) begrenzen.

2. Prothesenfuß nach Anspruch 1, dadurch gekennzeichnet, daß der obere Schenkel (2a) des Fußeinsatzes (2) in einer Horizontalebene gegenüber dem unteren Schenkel (2b) verwindbar ist, also eine Torsion und dadurch eine Rotation des Prothesenfußes um eine vertikale Achse zuläßt.

3. Prothesenfuß nach Anspruch 1 oder 2, dadurch gekennzeichnet, daß der obere Schenkel (2a) des Fußeinsatzes (2) in der lotrecht und quer zur Gehrichtung liegenden Frontalebene gegenüber dem unteren Schenkel (2b) verwindbar ist zur Anpassung an Neigungen quer zur Gehrichtung.

4. Prothesenfuß nach Anspruch 1, 2 oder 3, dadurch gekennzeichnet, daß die die Kraft-Weg- bzw. Winkel-Moment-Charakteristik der genannten Verschwenkungen bzw. Verformungen bestimmenden federelastischen Eigenschaften (Federkennlinien) durch den Z-förmigen Fußeinsatz (2) allein bestimmt sind. (Figuren 1, 2, 3)

5. Prothesenfuß nach Anspruch 1, 2 oder 3, dadurch gekennzeichnet, daß die genannten Federkennlinien im Zusammenwirken des Z-förmigen Fußeinsatzes (2) mit zusätzlichen federelastischen Komponenten (6,7,8) erzielt werden, die in zumindest einem der beiden genannten keilförmigen Freiräume (3,4) angeordnet sind.

6. Prothesenfuß nach Anspruch 5, dadurch gekennzeichnet, daß die zusätzlichen federelastischen Komponenten Druckfedern (7) sind. (Figur 5)

7. Prothesenfuß nach Anspruch 5, dadurch gekennzeichnet, daß die zusätzlichen federelastischen Komponenten durch einen zumindest einen der beiden Freiräume (3,4) ausfüllenden federelastischen Stoff (6) gebildet sind. (Figur 4)

8. Prothesenfuß nach Anspruch 4 oder 5, dadurch gekennzeichnet, daß separate oder integrierte Stützkonturen (8) an dem Z-förmigen Fußeinsatz vorgesehen sind. (Figur 6)

9. Prothesenfuß nach Anspruch 8, dadurch gekennzeichnet, daß sich durch die Stützkonturen (8) ein Anlagepunkt zwischen zwei Schenkeln (2b,2c) bei zunehmender Belastung verlagert.

10. Prothesenfuß nach einem der vorhergehenden Ansprüche, dadurch gekennzeichnet, daß die untere Kontur des unteren horizontalen Z-Schenkels (2b) an die Kontur der Fußunterseite angepaßt ist.

11. Prothesenfuß nach einem der vorhergehenden Ansprüche, gekennzeichnet durch eine den Verschwenkbereich des oberen und/oder unteren Z-Schenkels (2a,2b) im Sinne einer Vergrößerung des oberen bzw. unteren Winkels (α , β) begrenzende Einrichtung (9). (Figur 7)

12. Prothesenfuß nach einem der vorhergehenden Ansprüche, dadurch gekennzeichnet, daß der Fußeinsatz (2) ungegliedert einstückig ausgebildet ist. (Figuren 1, 4, 6, 7)

13. Prothesenfuß nach einem der Ansprüche 1 bis 10, dadurch gekennzeichnet, daß der Fußeinsatz (2) zweigliedrig ausgebildet ist, wobei sein oberer Schenkel (2a) mit dem oberen Ende des schrägen Z-Balkens (2c) verbunden ist. (Figur 2)

14. Prothesenfuß nach einem der Ansprüche 1 bis 10, dadurch gekennzeichnet, daß der Fußeinsatz (2) dreigliedrig ausgebildet ist, wobei seine beiden Schenkel (2a,2b) mit dem schrägen Z-Balken (2c) verbunden sind. (Figur 3)

15. Prothesenfuß nach einem der vorhergehenden Ansprüche, dadurch gekennzeichnet, daß der Prothesenkörper (1) aus einer alle Funktionselemente einbettenden fußförmigen Umschäumung besteht.

16. Prothesenfuß nach einem der vorhergehenden Ansprüche, dadurch gekennzeichnet, daß der Prothesenkörper (1) eine alle Funktionselemente umschließende kosmetische Hülle bildet.

## Claims

1. Non-jointed prosthetic foot with a resilient foot insert (2) which is provided within the body (1) of the prosthesis and receives and transfers the prosthesis loads, and which has an upper, horizontal section (2a) which forms the upper termination of the prosthetic foot and provides a possibility for connection to the prosthesis, as well as a lower section (2b) which is elongated with respect to the upper section (2a) and with its free end extends into the toe section of the prosthetic foot in order to achieve high elasticity in the front region of the foot in conjunction with a high energy storage capacity when there is load on the front region of the foot, characterized in that the foot insert (2), which allows at least a plantar and dorsal flexion as well as an axial compression, has an approximately Z-shaped construction in the longitudinal cross-section of the foot, the said upper section (2a) as well as the said lower section (2b) enclosing with the oblique bar (2c) of the Z an upper and a lower angle ($\alpha$, $\beta$) respectively and delimiting a rear and a front wedge-shaped free space (3, 4) respectively.

2. Prosthetic foot according to Claim 1, characterized in that the upper section (2a) of the foot insert (2) is twistable with respect to the lower section (2b) in a horizontal plane, and thus allows torsion and consequently rotation of the prosthetic foot about a vertical axis.

3. Prosthetic foot according to Claim 1 or 2, characterized in that the upper section (2a) of the foot insert (2) is twistable with respect to the lower section (2b) in the frontal plane located perpendicularly and transversely with respect to the walking direction in order to adapt to inclinations transverse with respect to the walking direction.

4. Prosthetic foot according to Claim 1, 2 or 3, characterized in that the resilient properties (spring characteristics) determining the forcedisplacement or angle/moment characteristic of the said tiltings or deformations are determined solely by the Z-shaped foot insert (2). (Figures 1, 2, 3)

5. Prosthetic foot according to Claim 1, 2 or 3, characterized in that the said spring characteristics are achieved by the cooperation of the Z-shaped foot insert (2) with additional resilient components (6, 7, 8) which are arranged in at least one of the two said wedge-shaped free spaces (3, 4).

6. Prosthetic foot according to Claim 5, characterized in that the additional resilient components are pressure springs (7). (Figure 5)

7. Prosthetic foot according to Claim 5, characterized in that the additional resilient components are formed by a resilient material (6) filling at least one of the two free spaces (3, 4). (Figure 4)

8. Prosthetic foot according to Claim 4 or 5, characterized in that separate or integrated support contours (8) are provided on the Z-shaped foot insert. (Figure 6)

9. Prosthetic foot according to Claim 8, characterized in that by means of the support contours (8) a bearing point between two sections (2b, 2c) is displaced with increasing load.

10. Prosthetic foot according to one of the preceding claims, characterized in that the lower contour of the lower horizontal section (2b) of the Z is matched to the contour of the underside of the foot.

11. Prosthetic foot according to one of the preceding claims, characterized by a means (9) delimiting the pivot region of the upper and/or lower section (2a, 2b) of the Z so as to enlarge the upper and lower angle ($\alpha$, $\beta$) respectively. (Figure 7)

12. Prosthetic foot according to one of the preceding claims, characterized in that the foot insert (2) is of undivided, one-piece construction. (Figures 1, 4, 6, 7)

13. Prosthetic foot according to one of Claims 1 to 10, characterized in that the foot insert (2) is of two-member construction, its upper section (2a) being connected to the upper end of the oblique bar (2c) of the Z. (Figure 2)

14. Prosthetic foot according to one of Claims 1 to 10, characterized in that the foot insert (2) is of three-member construction, its two sections (2a, 2b) being connected to the oblique bar (2c) of the Z. (Figure 3)

15. Prosthetic foot according to one of the preceding claims, characterized in that the body (1) of the prosthesis comprises a foot-shaped foam material in which all the functional elements are embedded.

**16.** Prosthetic foot according to one of the preceding claims, characterized in that the body (1) of the prosthesis forms a cosmetic sheath enclosing all the functional elements.

## Revendications

**1.** Pied de prothèse non articulé, comportant un insert de pied (2) élastique comme un ressort, qui est prévu à l'intérieur du corps de prothèse (1) et qui reçoit et transmet les charges de la prothèse, cet insert comprenant une branche supérieure horizontale (2a) qui constitue le bord supérieur du pied de prothèse et qui offre une possibilité de liaison avec la prothèse, ainsi qu'une branche inférieure (2b) formée avec un prolongement par rapport à la branche supérieure (2a) et qui s'étend par son extrémité libre jusque dans la région de la pointe du pied afin d'obtenir une grande élasticité de la partie antérieure du pied combinée à une grande capacité à accumuler l'énergie en cas de sollicitation de ladite partie antérieure du pied, caractérisé en ce que l'insert de pied (2), qui permet au moins une flexion plantaire et dorsale ainsi qu'une compression axiale, présente suivant une coupe longitudinale du pied une structure sensiblement en forme de Z, ladite branche supérieure (2a) et ladite branche inférieure (2b) formant respectivement avec la barre oblique (2c) du Z un angle supérieur ou inférieur (α, β) et délimitant respectivement avec cette barre un espace libre arrière ou avant (3, 4) en forme de coin.

**2.** Pied de prothèse conforme à la revendication 1, caractérisé en ce que la branche supérieure (2a) de l'insert de pied (2) est susceptible de se tordre dans un plan horizontal par rapport à la branche inférieure (2b) et permet ainsi une torsion et donc une rotation du pied de prothèse autour d'un axe vertical.

**3.** Pied de prothèse conforme à l'une des revendications 1 ou 2, caractérisé en ce que la branche supérieure (2a) de l'insert de pied (2) est susceptible de se tordre par rapport à la branche inférieure (2b) dans le plan frontal vertical perpendiculaire au sens de la marche, pour s'adapter à des inclinaisons perpendiculaires au sens de la marche.

**4.** Pied de prothèse conforme à l'une des revendications 1, 2 ou 3, caractérisé en ce que les propriétés élastiques du type ressort (caractéristiques de ressort) définissant la caractéristique force-déplacement ou moment angulaire desdits pivotements ou déformations sont déterminées par l'insert de pied (2) en forme de Z seul (figures 1, 2, 3).

**5.** Pied de prothèse conforme à l'une des revendications 1, 2 ou 3, caractérisé en ce que lesdites caractéristiques de ressort sont obtenues par la coopération de l'insert de pied (2) en forme de Z et de composants élastiques comme des ressorts supplémentaires (6, 7, 8) qui sont disposés dans au moins l'un desdits deux espaces libres en forme de coins (3, 4).

**6.** Pied de prothèse conforme à la revendication 5, caractérisé en ce que les composants élastiques comme des ressorts supplémentaires sont des ressorts de pression (7) (figure 5).

**7.** Pied de prothèse conforme à la revendication 5, caractérisé en ce que les composants élastiques comme des ressorts supplémentaires sont constitués par un matériau (6) élastique comme un ressort qui remplit au moins l'un des deux espaces libres (3, 4) (figure 4).

**8.** Pied de prothèse conforme à la revendication 4 ou 5, caractérisé en ce que des profils d'appui (8) séparés ou intégrés sont prévus sur l'insert de pied en forme de Z (figure 6).

**9.** Pied de prothèse conforme à la revendication 8, caractérisé en ce que, du fait des profils d'appui (8), un point d'application se déplace entre deux branches (2b, 2c) quand la charge augmente.

**10.** Pied de prothèse conforme à l'une des revendications précédentes, caractérisé en ce que le profil inférieur de la branche horizontale inférieure (2b) du Z est adapté au profil de la face inférieure du pied.

**11.** Pied de prothèse conforme à l'une des revendications précédentes, caractérisé par un dispositif (9) limitant la plage de pivotement de la branche supérieure et/ou inférieure (2a, 2b) du Z en vue d'augmenter l'angle supérieur ou inférieur (α, β) (figure 7).

**12.** Pied de prothèse conforme à l'une des revendications précédentes, caractérisé en ce que l'insert de pied (2) est réalisé d'une seule pièce sans élément articulé (figures 1, 4, 6, 7).

**13.** Pied de prothèse conforme à l'une des revendications 1 à 10, caractérisé en ce que l'insert de pied (2) est réalisé en deux parties articulées, sa branche supérieure (2a) étant reliée à l'extrémité supérieure de la barre oblique (2c) du Z (figure 2).

**14.** Pied de prothèse conforme à l'une des revendications 1 à 10, caractérisé en ce que l'insert de pied (2) est réalisé en trois parties articulées, ses deux branches (2a, 2b) étant reliées à la barre oblique (2c) du Z (figure 3).

**15.** Pied de prothèse conforme à l'une des revendications précédentes, caractérisé en ce que le corps de prothèse (1) est un élément en mousse en forme de pied enrobant tous les éléments fonctionnels.

**16.** Pied de prothèse conforme à l'une des revendications précédentes, caractérisé en ce que le corps de prothèse (1) constitue une enveloppe esthétique entourant tous les éléments fonctionnels.

Fig. 1

Fig.2

2a

α +

1

2c

β

2b

EP 0 401 864 B1

Fig.3

EP 0 401 864 B1

Fig. 4

Fig. 5

EP 0 401 864 B1

Fig. 6

EP 0 401 864 B1

Fig. 7

EP 0 401 864 B1